# EUROPEAN PATENT SPECIFICATION

(11) **EP 3 777 804 B1**
(45) Date of publication and mention of the grant of the patent: **22.06.2022**
(21) Application number: 18911488.7
(22) Date of filing: 12.04.2018
(51) Int. Cl.: A61F 13/532

(54) **ABSORBENT ARTICLE**
ABSORBIERENDER ARTIKEL
ARTICLE ABSORBANT

(30) Priority: 28.03.2018 JP 2018062380
(43) Date of publication of application: 17.02.2021
(73) Proprietor: Unicharm Corporation, Shikokuchuo-shi, Ehime 799-0111 (JP)
(72) Inventor: SHIMAZU, Takeshi, Kanonji-shi, Kagawa 769-1602 (JP); NAKAO, Hitomi, Kanonji-shi, Kagawa 769-1602 (JP); MIYAZAKI, Hirokazu, Kanonji-shi, Kagawa 769-1602 (JP); MIYAMAE, Naomu, Kanonji-shi, Kagawa 769-1602 (JP)
(74) Representative: Staeger & Sperling Partnerschaftsgesellschaft mbB
(86) International application number: PCT/JP2018/015360
(87) International publication number: WO 2019/187173

(56) References cited:
- EP-A1- 2 438 893
- WO-A1-2013/035317
- JP-A- 2002 524 148
- JP-A- 2004 049 764
- JP-A- 2008 161 256
- JP-A- 2009 201 848
- US-A1- 2019 076 307

## Description

### [Technical Field]

The present invention relates to an absorbent article such as a disposable diaper.

### [Background Art]

Patent Literature 1 discloses an absorbent article in which an absorbent core is narrowly formed in accordance with a width of a crotch of a wearer. The absorbent article of Patent Literature 1 has an upper layer having a top sheet and an absorbent core, and a lower layer having a back sheet and an elastic member. In an intermediate portion positioned midway in the longitudinal direction, the upper layer is divided into three portions in the width direction by a slit or the like, and has a central portion and a pair of side portions. The elastic member is contractible in the longitudinal direction and is disposed outside the central portion in the width direction. Due to the contraction of the elastic member, the outside in the width direction is raised from the central portion, and the side portion moves inward in the width direction. As a result, the length of an absorbent core in the width direction is shortened.

### [Patent Literature]

Patent Literature 1: Japanese Unexamined Patent Publication No. 2012-16414

### Summary of the Invention

However, in the absorbent article of Patent Literature 1, an inner portion of the side portion in the width direction is not bonded to the lower layer, and a portion of the side portion that is not bonded to the lower layer moves inward in the width direction. Further, in the absorbent article of Patent Literature 1, an outer portion of the side portion in the width direction is bonded to the lower layer, and a portion of the side portion that is bonded to the lower layer is unlikely to move inward in the width direction. Therefore, the length of the absorbent core in the width direction cannot be sufficiently shortened, and it has been demanded to further improve the comfort at the time of wearing.

Therefore, an absorbent article which can sufficiently shorten the length of the absorbent core in the width direction and can further improve the comfort at the time of wearing is desired.

According to one aspect of the present invention, there is provided an absorbent article includes a longitudinal direction and a width direction orthogonal to each other; a crotch region including a center in the longitudinal direction; a front side region positioned on a front side of the crotch region, and a rear side region positioned on a rear side of the crotch region; and an absorbent core disposed at least in the crotch region, in which the absorbent core has a pair of side slits formed to extend in the longitudinal direction, and the absorbent core includes a central region sandwiched by the side slits and side regions positioned between the side slit and outer edge of the absorbent core, the side slit includes a wide portion having a length in the width direction equal to or greater than a length in the width direction of the side region in the crotch region, and a center of the wide portion in the longitudinal direction is positioned on a front side from a center of the absorbent article in the longitudinal direction.

### [Brief Description of Drawings]

Fig. 1 is a plan view of an absorbent article according to an embodiment when viewed from a skin surface side.
Fig. 2 is a plan view of an absorbent core according to an embodiment when viewed from a skin surface side.
Fig. 3 is a cross-sectional view taken along an A-A line illustrated in Fig. 1.
Fig. 4 is a diagram illustrating a modification of the absorbent core.

### [Description of Embodiments]

### (1) SUMMARY OF THE EMBODIMENTS

Description of the present specification and accompanying drawings clarify at least the following matters.

According to one aspect of the present invention, there is provided an absorbent article includes a longitudinal direction and a width direction orthogonal to each other; a crotch region including a center in the longitudinal direction; a front side region positioned on a front side of the crotch region, and a rear side region positioned on a rear side of the crotch region; and an absorbent core disposed at least in the crotch region, in which the absorbent core has a pair of side slits formed to extend in the longitudinal direction, and the absorbent core includes a central region sandwiched by the side slits and side regions positioned between the side slit and outer edge of the absorbent core, the side slit includes a wide portion having a length in the width direction equal to or greater than a length in the width direction of the side region in the crotch region, and a center of the wide portion in the longitudinal direction is positioned on a front side from a center of the absorbent article in the longitudinal direction.

Since the length of the wide portion of the side slit in the width direction is equal to or greater than the length of the side region in the width direction, the side slit can enter inward in the width direction over the entire side region in the width direction. Since the length of the absorber in the width direction is shortened, the absorbent core is easily accommodated in the crotch, and uncomfortable feeling at the time of wearing can be suppressed. The center of the wide portion in the longitudinal direction is positioned on the front side of the center of the absorbent article in the longitudinal direction, and it is likely to obtain the effect of shortening the length of the absorber in the width direction on the front side of the absorbent article. Therefore, the wearing feeling can be improved even in the sitting posture in which the crotch width is narrowed.

According to a preferable aspect, the rear edge of the side slit may be positioned in the crotch region.

According to the present aspect, the rear edge of the side slit is positioned within the crotch region, the side slit does not reach the rear side region. Therefore, a region in which the length of the absorber in the width direction is shortened hardly reaches the rear side region, and the absorber in the rear side region can be applied to the body with a wide width to suppress leakage.

According to a preferable aspect, the side slit may be positioned inside the outer edge of the absorbent core in the width direction.

According to the present aspect, when a side joint portion is teared inward in the longitudinal direction from the outer edge of the side joint portion in the longitudinal direction, it is possible to immediately reach a region, in which a hydrophilic sheet is disposed, easily teared . Therefore, the user can easily tear the side joint portion.

According to a preferable aspect, the absorbent article further includes a top sheet positioned on a skin surface side of the absorber; and an erecting leakage-preventing gather positioned on a skin surface side of the top sheet, in which the leakage-preventing gather includes a contraction portion erected by contraction of a leakage-preventing elastic member and a base point portion serving as a base point of rise of the contraction portion, and the base point portion positioned outside the contraction portion in the width direction may overlap the side region in a thickness direction.

According to the present aspect, the base point portion positioned outside the contraction portion in the width direction is pulled inward in the width direction and toward the skin surface side by contraction of the contraction portion. Since the base point portion overlaps the side region in the thickness direction, a force is generated to pull the side region inward in the width direction and toward the skin surface side. When a force from the outside to the inside in the width direction is applied at the time of wearing, the side region tends to move toward the skin surface side of the central region and inward in the width direction. Therefore, the length of the absorbent core in the width direction is likely to be shortened.

According to a preferable aspect, a leg elastic member contracting in the longitudinal direction may be provided outside the side region in the width direction.

According to the present aspect, the side region is pulled up to the skin surface side by the contraction of the leg elastic member, and when a force from the outside to the inside in the width direction is applied at the time of wearing, the side region tends to move toward the skin surface side of the central region and inward in the width direction. Therefore, the length of the absorbent core in the width direction is likely to be shortened.

According to a preferable aspect, on a virtual line extending in the width direction across the wide portion, a length of the side region in the width direction may be longer than a length of the central region in the width direction.

According to the present aspect, when the side region moves inward in the width direction and toward the skin surface side of the central region, the central region and the side region overlap each other, and the length of the entire absorbent core in the width direction can be shortened. Since the length of the side region in the width direction is longer than the length of the central region in the width direction, the side region can be easily accommodated within the range of the central region in the width direction, and the length of the entire absorbent core in the width direction can be shortened.

According to a preferable aspect, on a virtual line extending in the width direction across the wide portion, a length of the side region in the width direction may be equal to or less than 1/2 of a length of the central region in the width direction.

According to the present aspect, when the side region moves inward in the width direction and toward the skin surface side of the central region, the central region overlaps the side region. At this time, since the length of the side region in the width direction is equal to or less than 1/2 of the length of the central region in the width direction, even if the entire side region is moved inward in the width direction so as to overlap the central region, it is possible to suppress the overlapping of the side regions. Therefore, it is possible to suppress an increase in the local thickness due to the overlapping of the side regions, and to suppress a decrease in the wearing feeling.

According to a preferable aspect, the absorbent core may be provided with a central slit positioned between the side slits in the width direction and extending in the longitudinal direction.

According to the present aspect, the central region is likely to be bent in the thickness direction by the central slit, and the length of the entire absorber in the width direction can be made shorter.

According to a preferable aspect, the absorbent core may include a constricted portion having a length in the width direction shorter than a maximum width of the absorbent core, and the constricted portion may be disposed in the crotch region.

According to the present aspect, since the length of the absorbent core in the width direction in the crotch region sandwiched between the crotches of the wearer is short, the absorbent core is more likely to be accommodated in the crotch of the wearer, and the uncomfortable feeling at the time of wearing can be suppressed.

According to a preferable aspect, a center of the constricted portion in the longitudinal direction may be positioned on a front side of the center of the absorbent article in the longitudinal direction.

According to the present aspect, it is likely to obtain an effect of shortening the length of the absorber in the width direction on the front side of the absorbent article. Therefore, the wearing feeling can be improved even in the sitting posture in which the crotch width is narrowed.

According to a preferable aspect, a front edge of the side slit may be positioned on a front side of a front edge of the constricted portion, and a rear edge of the side slit may be positioned on a front side of a rear edge of the constricted portion.

According to the present aspect, by forming a deformation base point by the side slit from the front side of the constricted portion toward the rear side, it is possible to improve the wearing feeling even in a sitting posture. Further, the rear edge of the side slit is positioned on the rear side of the rear edge of the constricted portion, and thus it is possible to suppress the leakage by applying the absorbent core to the body in the rear side than the side slit.

According to a preferable aspect, the wide portion may be disposed in a region where the constricted portion is provided in the longitudinal direction.

According to the present aspect, since the absorbent core is sandwiched by the legs, the length of the absorbent core at the constricted portion in the width direction is shortened by deformation of the wide portion. The absorbent core is more likely to be accommodated in the crotch space, and thus it is possible to suppress the uncomfortable feeling at the time of wearing.

According to a preferable aspect, an inner edge of the side slit may extend outward in the width direction from the wide portion toward a rear side.

According to the present aspect, since the inner edge of the side slit extends outward in the width direction from the wide portion toward the rear side, the width of the central region sandwiched by the side slits becomes longer toward the rear side from the wide portion. The region between the side slits that can be easily placed against the body can be provided wide toward the rear side, and the absorbent core can be applied to the buttocks to suppress the leakage.

### (2) Embodiment of absorbent article

Hereinafter, a disposable diaper according to an embodiment will be described with reference to drawings. In the following description of the drawings, identical or similar portions will be given an identical or similar reference sign. The drawings are schematic, and attention needs to be paid to the fact that the ratio and the like of individual dimensions differ from the actual ones. Therefore, specific dimensions and the like need to be determined with reference to the following reference. In addition, dimensional relationships or ratios between portions are not always identical among the drawings.

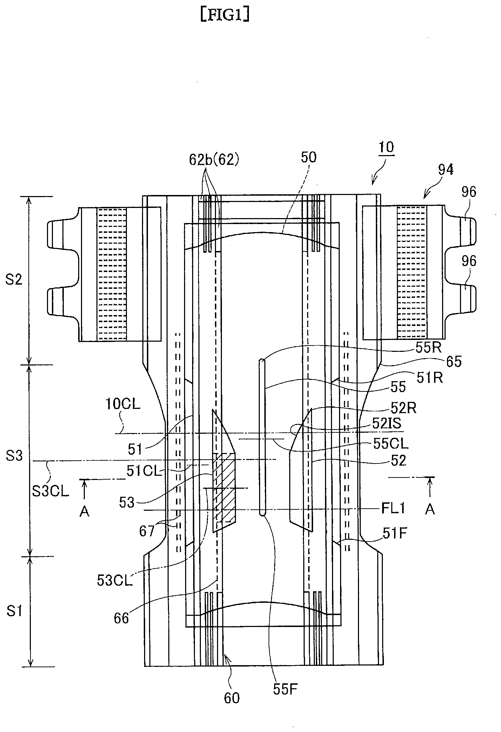

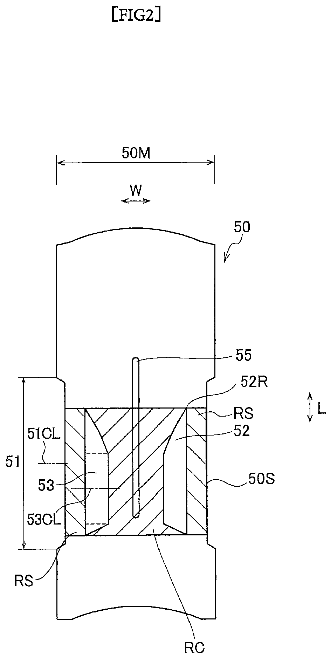

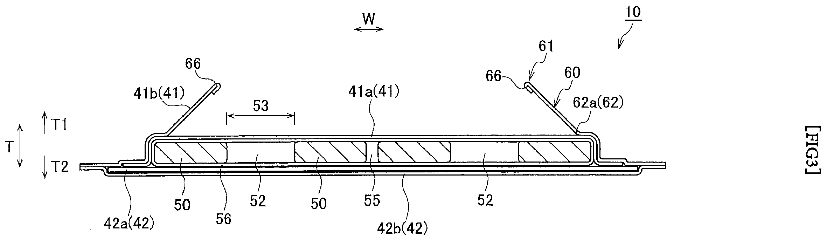

Fig. 1 is a plan view of an absorbent article 10 according to an embodiment when viewed from a skin surface side, and Fig. 2 is a plan view of an absorbent core 50 according to an embodiment when viewed from a skin surface side. The plan views illustrated in Figs. 1 and 2 illustrate an elongated state in which the absorbent article 10 is stretched to a state in which wrinkles are not formed. Fig. 3 is a cross-sectional view taken along an A-A line illustrated in Fig. 1. The absorbent article 10 of the present embodiment is a deployable adult absorbent article, but is not limited thereto. The absorbent article 10 may be a pant-type absorbent article or an absorbent article for children.

An absorbent article 10 has a longitudinal direction L and a width direction W orthogonal to each other. The longitudinal direction L is defined by a direction extending to the front side of the body and the rear side of the body. In other words, the longitudinal direction L is a direction extending back and front in the expanded absorbent article 10. In addition, the absorbent article 10 has a thickness direction T orthogonal to both the longitudinal direction L and the width direction W. The thickness direction T extends to the skin surface side T1 toward the wearer side and the non-skin surface side T2 away from the wearer. In the present specification, the "skin surface side" corresponds to the side facing the wearer's skin during use. The "non-skin surface side" corresponds to the side facing the side opposite to the wearer's skin during use.

The absorbent article 10 has a front side region S1, a rear side region S2, and a crotch region S3. The front side region S1 faces the front side of the wearer's body when in use. The rear side region S2 faces the rear side of the wearer's body when in use. The crotch region S3 is positioned between the front side region S1 and the rear side region S2, and is disposed at the wearer's crotch when in use. The crotch region S3 is a region in which a leg opening 65 is provided, the boundary between the front side region S1 and the crotch region S3 may be defined by the front edge of the leg opening 65, and the boundary between the rear side region S2 and the crotch region S3 may be defined by the rear edge of the leg opening 65. The leg opening 65 is a portion recessed inward in the width direction W at an outer edge of a main body portion of the absorbent article (which is a portion of the absorbent article excluding a fastening tape 94 to be described later). Note that, the outer edge of the present embodiment is an edge on the outside in the width direction, and the inner edge is an edge on the inside in the width direction.

The absorbent article 10 includes the absorbent core 50 disposed in at least the crotch region S3. The absorbent core 50 may extend from the crotch region S3 to the front side region S1 and the rear side region S2. The absorbent core 50 may be a laminate of absorbent materials including, for example, a ground pulp or a superabsorbent polymer (SAP), or a mixture thereof. The absorbent article 10 may include an absorber that includes an absorbent core 50 and a core wrap 56. The core wrap 56 is disposed so as to cover the skin surface side and the non-skin surface side of the absorbent core 50, and may be, for example, a tissue or a non-woven fabric sheet. The configuration of the absorbent core will be described below in detail.

The absorbent article 10 may include a skin surface sheet 41 positioned on the skin surface side T1 of the absorbent core 50 and a non-skin surface sheet 42 positioned on the non-skin surface side T2 of the absorbent core 50. The skin surface sheet 41 may include a top sheet 41a and a side sheet 41b. The top sheet 41a has liquid permeability and may be disposed so as to cover at least the center of the absorbent core 50 in the width direction W. The side sheet 41b is made of a non-woven fabric having hydrophobicity, and may be disposed so as to cover both side portions of the top sheet 41a in the width direction W outside the center of the absorbent core 50 in the width direction W. The non-skin surface sheet 42 has liquid impermeability and may be provided on the non-skin surface side of the absorbent core 50. The non-skin surface sheet 42 may include a liquid-impermeable back film 42a and a back non-woven fabric 42b disposed on the non-skin surface side T2 of the back film 42a.

The absorbent article 10 may include an erecting leakage-preventing gather 60. The leakage-preventing gather 60 is positioned on the skin surface side of the top sheet 41a and can erect with respect to the top sheet 41a. The leakage-preventing gather 60 includes a contraction portion 61 erecting by contraction of a leakage-preventing elastic member 66, and a base point portion 62 serving as a base point of rise of the contraction portion 61. The base point portion 62 may include a first base point portion 62a (refer to Fig. 2) positioned outside the contraction portion 61 in the width direction W and a second base point portion 62b (refer to Fig. 1) positioned outside the contraction portion 61 in the longitudinal direction L. The leakage-preventing elastic member 66 is disposed on the inner edge of the side sheet 41b, to erect the inner edge of the side sheet 41b on the wearer side.

The absorbent article 10 may include a leg elastic member 67 disposed in the leg opening 65. The leg elastic member 67 is disposed along leg opening 65 to fit the leg opening 65 around the wearer's leg. The leg elastic member 67 is disposed between the side sheet 41b and the back film 42a. The leakage-preventing elastic member 66 and the leg elastic member 67 are expandable and contractible in the longitudinal direction L, and may be arranged along the longitudinal direction L.

The absorbent article 10 may include fastening tapes 94 provided on both outer portions of the rear side region S2 in the width direction W. The fastening tape 94 may include an engaging portion 96 that engages with the front side region S1.

Next, the configuration of the absorbent core 50 will be described in detail. A slit may be formed in the absorbent core 50. The slit is a portion in which the absorbent material forming the absorbent core 50 is not substantially disposed. The slit is a portion where a basis weight of the absorbent material is 0 or an absorbent material which has been spilled from the periphery is disposed, but the designed absorbent material is 0. When the absorbent core 50 is subjected to a force, the slit absorbs deformation or serves as a base point for deformation. The slit may include a pair of side slits 52 and a central slit 55.

As illustrated in Fig. 2, the absorbent core 50 may include a central region RC sandwiched by the side slits 52 and a side region RS positioned between the side slit 52 and the outer edge 50S of the absorbent core 50. In Fig. 2, the central region RC and the side region RS are indicated with different hatched lines. A pair of the side regions RS are positioned and provided outside each side slit 52 in the width direction W. The central region RC and the side region RS are regions extending along the width direction W from the side slit 52. At least a part of the central slit 55 may be disposed in the central region RC.

The side slit 52 may be disposed at least in the crotch region S3. The front edge of the side slit 52 may be disposed in the front side region S1 or may be disposed in the crotch region. The rear edge 52R of the side slit 52 may be disposed in the crotch region S3. The side slit 52 may extend at least in the longitudinal direction L, and may be along the longitudinal direction L or may include a portion inclined with respect to the longitudinal direction L. The length of the side slit 52 in the width direction W may be constant or may be changed. The side slits 52 may be provided symmetrically in pairs with respect to the center of the absorbent core 50 in the width direction W.

The side slit 52 includes a wide portion 53. The wide portion 53 is a portion having a length in the width direction W which is longer than the length of the side region RS in the width direction W. The length of the wide portion 53 in the width direction W may be equal to or greater than the length of the side region RS adjacent to the wide portion 53 in the width direction W, in an extended state of the absorbent article 10. At least a part of the wide portion 53 may be disposed in the crotch region S3, and the entire region of the wide portion 53 in the longitudinal direction L may be disposed in the crotch region S3. The front edge of the wide portion 53 may be disposed in the crotch region S3. A center 53CL of the wide portion 53 in the longitudinal direction L is positioned on the front side of a center 10CL of the absorbent article 10 in the longitudinal direction L, and may be positioned on the front side of the center of the crotch region S3 in the longitudinal direction L.

The side slit 52 is positioned on the inner side of the outer edge 50S of the absorbent core 50 in the width direction, and does not need to reach the outer edge 50S of the absorbent core 50. According to this configuration, the absorbent material exists outside the side slit 52 in the width direction W. A body fluid diffused by the side slit 52 is held by the absorbent material, and lateral leakage can be suppressed.

The central slit 55 may be positioned between the side slits 52 in the width direction W and extend in the longitudinal direction L. At least a part of the central slit 55 may be disposed in the crotch region S3. A front edge 55F of the central slit 55 is disposed in the crotch region S3, and the rear edge 55R of the central slit 55 may terminate in the crotch region S3. The central slit 55 may extend at least in the longitudinal direction L, and may be along the longitudinal direction L or may include a portion inclined with respect to the longitudinal direction L. A center 55CL of the central slit 55 in the longitudinal direction L is positioned on the front side of the center of the absorbent article 10 in the longitudinal direction L, and may be positioned on the rear side of the center S3CL of the crotch region S3 in the longitudinal direction L. The central slit 55 may be biased on the rear side of the side slit 52.

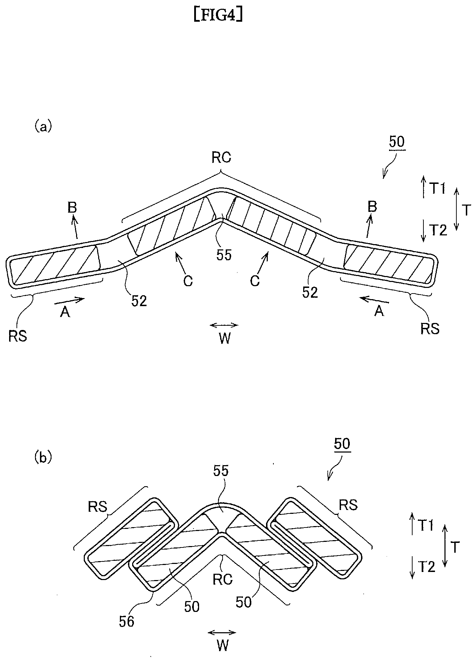

Next, a modification of the absorbent article 10 configured as described above will be described. Fig. 4 is a diagram schematically illustrating a modification of the absorber at the time of wearing. Fig. 4 schematically illustrates the modification of the absorber in the cross-section illustrated in Fig. 3. When the absorbent article 10 is sandwiched between both legs in a state in which the absorbent article 10 is worn, a force directed inward in the width direction W is applied to the crotch region S3 by the legs. When a force directed inward is applied to the absorber of the crotch region S3 in the width direction W, the side slit 52 of the absorbent core 50 is deformed, the side region RS moves inward in the width direction W (arrow A in Fig. 4 (a)), and the length of the absorber in the width direction W is shortened.

At this time, a maximum length that the side region RS can be moved inward in the width direction W is a maximum length of the side slit 52 the width direction W, that is, the length of the wide portion 53 in the width direction W. Since the length of the wide portion 53 of the side slit 52 in the width direction W is equal to or greater than the length of the side region RS in the width direction W, the side slit can enter inward in the width direction over the entire side region RS in the width direction. Since the length of the absorber in the width direction is shortened, the absorbent core 50 is easily accommodated in the crotch space, and uncomfortable feeling at the time of wearing can be suppressed.

The width of the crotch of the wearer varies with posture, and becomes narrower in the order of a supine posture, a sideways lying posture, and a sitting posture. In particular, in the sitting posture, the force by the legs is likely to be applied to the front side of the absorbent article 10. The center of the wide portion 53 in the longitudinal direction L is positioned on the front side of the center of the absorbent article 10 in the longitudinal direction L, and it is likely to obtain the effect of shortening the length of the absorber in the width direction on the front side of the absorbent article 10. Therefore, the wearing feeling can be improved even in the sitting posture in which the crotch width is narrowed.

If the absorber is not accommodated in the crotch space in the sitting posture and the uncomfortable feeling occurs, comfort may be impaired, or the sitting position may be shifted in a posture of leaning back. More specifically, in the sitting posture, it is likely to maintain a stable posture in a state in which the legs are closed, and in a state in which the legs are difficult to close (a state in which the legs are open), the center of gravity tilts backward and rests against a backrest of a chair or the like, and a shift in the sitting position is likely to occur. According to the absorbent article of the present embodiment, since the absorber is likely to be accommodated in the crotch in the sitting posture, comfort is improved and it is likely to maintain an appropriate sitting posture.

Note that, as illustrated in Fig. 4 (a), regarding the deformation of the absorber due to the absorbent article 10 sandwiched by both legs, the absorber may be deformed into a convex shape having the center of the absorber in the width direction as an apex, and the side region RS may slide inward in the width direction without deforming the absorber in the thickness direction. Even in the deformation in which the side region RS slides, the length of the absorbent core in the width direction is shortened by the length of the side slit 52 in the width direction.

The rear edge 52R of the side slit 52 is positioned within the crotch region S3, the side slit 52 may not reach the rear side region S2. According to this configuration, the region in which the length of the absorber in the width direction W is shortened hardly reaches the rear side region S2, and the absorbent core 50 in the rear side region S2 can be applied to the body with a wide width to suppress leakage.

The first base point portion 62a, which is positioned outside the contraction portion 61 in the width direction W, of the base point portions 62 constituting the leakage-preventing gather 60 may overlap with the side region RS in the thickness direction T. The first base point portion 62a positioned outside the contraction portion 61 in the width direction W is pulled inward in the width direction W and toward the skin surface side T1 by contraction of the contraction portion 61. Since the first base point portion 62a overlaps the side region RS in the thickness direction T, a force is generated to pull the side region RS inward in the width direction W and toward the skin surface side T1. When the applied force at the time of wearing, the side region RS is pulled to the skin surface side T1 (arrow B from the outside to the inside in the width direction of Fig. 4(a)) of the central region RC and to the inside in the width direction (arrow A of Fig. 4(a)). Therefore, the side region RS moves toward the skin surface side T1 of the central region RC and inward in the width direction W, and the length of the absorbent core 50 in the width direction tends to become shorter. In the state illustrated in Fig. 4(a), the side region RS moves further toward the skin surface side T1 of the central region RC and inward in the width direction W, and thereby the state illustrated in Fig. 4(b) is obtained. In the state illustrated in Fig. 4 (b), the central region RC and the side region RS overlap each other in the thickness direction, and the length of the absorber in the width direction becomes shorter.

In the extended state of the absorbent article as illustrated in Fig. 1, the inner edge of the contraction portion 61 of the leakage-preventing gather 60 may overlap the wide portion 53 of the side slit 52 in the thickness direction. Since the contraction portion 61 overlaps the wide portion 53 in the thickness direction T, a force is generated to pull the wide portion 53 inward in the width direction W and toward the skin surface side T1. When a force from the outside to the inside in the width direction is applied at the time of wearing, the side region RS is likely to be pulled toward the skin surface side of the central region RC and inward in the width direction, as indicated by an arrow B in Fig. 4(a). Therefore, the side region RS moves toward the skin surface side of the central region RC and inward in the width direction, and the length of the absorbent core 50 in the width direction tends to become shorter.

The leg elastic member 67 contracting in the longitudinal direction L may be provided outside the side region RS in the width direction. The side region RS is pulled up to the skin surface side T1 by the contraction of the leg elastic member 67, and when a force from the outside to the inside in the width direction is applied at the time of wearing, the side region RS tends to move toward the skin surface side T1 of the central region RC and inward in the width direction. Therefore, the length of the absorbent core 50 in the width direction is likely to be shortened.

On the virtual line (for example, FL1) extending in the width direction across the wide portion 53, the length of the side region RS in the width direction W may be longer than the length of the central region RC in the width direction W. When the side region RS moves inward in the width direction and toward the skin surface side T1 of the central region RC, the central region RC and the side region RS overlap each other, and the length of the entire absorbent core 50 in the width direction can be shortened. Since the length of the side region RS in the width direction is longer than the length of the central region RC in the width direction, the side region RS can be easily accommodated within the range of the central region RC in the width direction, and the length of the entire absorbent core 50 in the width direction can be shortened. Note that, the virtual line FL1 is a line extending along the width direction, and is a line disposed to overlap the wide portion 53. The virtual line FL1 may overlap at least a part of the region from the front edge to the rear edge of the wide portion 53.

More preferably, the virtual line FL1 extending in the width direction across the wide portion 53, the length of the side region RS in the width direction may be equal to or less than 1/2 of the length of the central region RC in the width direction. When the side region RS moves inward in the width direction and toward the skin surface side of the central region RC, the central region RC overlaps the side region RS. At this time, since the length of the side region RS in the width direction is equal to or less than 1/2 of the length of the central region RC in the width direction, even if the entire side region RS is moved inward in the width direction so as to overlap the central region RC, it is possible to suppress the overlapping of the side regions RS. Therefore, it is possible to suppress an increase in the thickness of the absorbent core locally due to the overlapping of the side regions RS. Therefore, the concentration of pressure due to the local increase in the thickness of the absorbent core is less likely to occur, and the deterioration of the wearing feeling can be suppressed.

In the absorbent core 50, a central slit 55 positioned between the side slits 52 in the width direction W, and extending in the longitudinal direction L may be formed. The central region RC is likely to be bent in the thickness direction by the central slit 55, and the length of the entire absorber in the width direction can be made shorter. More specifically, when a force from the outside to the inside in the width direction is applied at the time of wearing, the absorbent core 50 is likely to be deformed such that the inner edge of the central region RC is directed toward the skin surface side from the outer edge (the side slit 52) of the central region RC as indicated by an arrow C in Fig. 4(a). Therefore, the length of the absorbent core 50 in the width direction is more likely to be shortened.

The absorbent core 50 may include a constricted portion 51 whose length in the width direction W is shorter than the maximum width of the absorbent core 50. The region having the maximum width of 50 M of the absorbent core 50 is disposed over the front side region S1 and the crotch region S3, and is disposed over the rear side region S2 and the crotch region S3. The constricted portion 51 of the absorbent core 50 may be disposed in the crotch region S3. Since the length of the absorbent core 50 in the width direction W in the crotch region S3 sandwiched between the crotches of the wearer is short, the absorbent core 50 is likely to be accommodated in the crotch of the wearer, and the uncomfortable feeling at the time of wearing can be suppressed.

A center 51CL of the constricted portion 51 in the longitudinal direction L may be positioned on the front side of a center 10CL of the absorbent article 10 in the longitudinal direction L. It is likely to obtain an effect of shortening the length of the absorber in the width direction W on the front side of the absorbent article 10. Therefore, the wearing feeling can be improved even in the sitting posture in which the crotch width is narrowed.

The front edge of the side slit 52 may be positioned on the front side of the front edge 51F of the constricted portion 51, and the rear edge 52R of the side slit 52 may be positioned on the front side of the rear edge 51R of the constricted portion 51. The center 53CL of the wide portion 53 in the front and rear direction may be positioned on the front side of the center 51CL of the constricted portion 51 in the front and rear direction. In the sitting posture in which the crotch width is narrowed, the force by the leg is likely to be applied not only to the region (constricted portion) sandwiched by the legs, but also to the front side of the constricted portion 51. By forming a deformation base point by the side slit 52 from the front side of the constricted portion 51 to the rear side, it is possible to improve the wearing feeling even in a sitting posture. Further, the rear edge of the side slit 52 is positioned on the rearside of the rear edge of the constricted portion 51, and thus it is possible to suppress the leakage by applying the absorbent core 50 to the body in the rear side than the side slit 52.

The wide portion 53 may be disposed in a region where the constricted portion 51 is provided in the longitudinal direction L. Since the absorbent core 50 is sandwiched by the legs, the length of the absorbent core 50 at the constricted portion 51 in the width direction is shortened by deformation of the wide portion 53. The absorbent core 50 is more likely to be accommodated in the crotch space, and thus it is possible to suppress the uncomfortable feeling at the time of wearing.

An inner edge 52IS of the side slit 52 may extend outward in the width direction from the rear edge of the wide portion 53 toward the rear side. Since the inner edge of the side slit 52 extends outward in the width direction from the rear edge of the wide portion 53 toward the rear side, the width of the central region RC sandwiched by the side slits 52 becomes longer toward the rear side from the wide portion 53. The region between the side slits 52 that can be easily placed against the body can be provided wide toward the rear side, and the absorbent core 50 can be applied to the buttocks to suppress the leakage.

Hitherto, the present invention has been described in detail using the above-described embodiments, but it is clear to a person skilled in the art that the present invention is not limited to the embodiments described in the present specification. The present invention can be carried out as a correction and modification aspect within the gist and scope of the present invention determined by the description in claims. Therefore, the description of the present specification is intended for exemplary description and does not have any meaning limiting the present invention in any fashion.

The entire contents of Japanese Patent Application No. 2018-062380 (filed on March 28, 2018) is incorporated into the present specification by reference.

### [Industrial applicability]

It is possible to provide an absorbent article which can sufficiently shorten the length of the absorbent core in the width direction and can further improve the comfort at the time of wearing.

### [Reference Signs List]

10: Absorbent article
50: Absorbent core
51: Constricted portion
52: Side slit
52R: Rear edge of side slit
53: Wide portion
55: Central slit
60: leakage-preventing gather
61: Contraction portion
62a: First base point portion (base point portion)
67: leg elastic member
RC: Central region
RS: Side region
S1: front side region
S2: rear side region
S3: crotch region
L: Longitudinal direction
T: Thickness direction
T1: Skin surface side
T2: Non-skin surface side
W: Width direction

## Claims

1. An absorbent article (10) comprising:
a longitudinal direction (L) and a width direction (W) orthogonal to each other;
a crotch region (S3) including a center in the longitudinal direction;
a front side region (S1) positioned on a front side of the crotch region, and a rear side region (S2) positioned on a rear side of the crotch region; and
an absorbent core (50) disposed at least in the crotch region, wherein the absorbent core has a pair of side slits (52) formed to extend in the longitudinal direction, and the absorbent core includes a central region (RC) sandwiched by the side slits and side regions (RS) positioned between the side slits and outer edges of the absorbent core,
wherein the side slit includes a wide portion (53) having a length in the width direction equal to or greater than a length in the width direction of the side region in the crotch region, and
wherein a center (53CL) of the wide portion in the longitudinal direction is positioned on a front side from a center (10CL) of the absorbent article in the longitudinal direction.

2. The absorbent article according to claim 1,
wherein a rear edge (52R) of the side slit is positioned within the crotch region.

3. The absorbent article according to claim 1 or 2,
wherein the side slit is positioned inside the outer edge (50S) of the absorbent core in the width direction.

4. The absorbent article according to any one of claims 1 to 3, further comprising:
a top sheet (41a) positioned on a skin surface side of the absorbent core; and
an erecting leakage-preventing gather (60) positioned on a skin surface side of the top sheet,
wherein the leakage-preventing gather includes a contraction portion (61) erected by contraction of a leakage-preventing elastic member (66) and a base point portion (62) serving as a base point of rise of the contraction portion, and
wherein the base point portion positioned outside the contraction portion in the width direction overlaps the side region in a thickness direction(T) .

5. The absorbent article according to any one of claims 1 to 4,
wherein a leg elastic member (67) contracting in the longitudinal direction is provided outside the side region in the width direction.

6. The absorbent article according to any one of claims 1 to 5,
wherein on a virtual line (FL1) extending in the width direction across the wide portion, a length of the side region in the width direction is longer than a length of the central region in the width direction.

7. The absorbent article according to any one of claims 1 to 5,
wherein on a virtual line (FL1) extending in the width direction across the wide portion, a length of the side region in the width direction is equal to or less than 1/2 of a length of the central region in the width direction.

8. The absorbent article according to any one of claims 1 to 7,
wherein the absorbent core is provided with a central slit (55) positioned between the side slits in the width direction and extending in the longitudinal direction.

9. The absorbent article according to any one of claims 1 to 8,
wherein the absorbent core includes a constricted portion (51) having a length in the width direction shorter than a maximum width of the absorbent core, and
wherein the constricted portion is disposed in the crotch region.

10. The absorbent article according to claim 9,
wherein a center (51CL) of the constricted portion in the longitudinal direction is positioned on a front side of the center of the absorbent article in the longitudinal direction.

11. The absorbent article according to claim 9 or 10,
wherein a front edge of the side slit is positioned on a front side of a front edge of the constricted portion, and
a rear edge (52R) of the side slit is positioned on a front side of a rear edge (51R) of the constricted portion.

12. The absorbent article according to any one of claims 9 to 11,
wherein the wide portion is disposed in a region where the constricted portion is provided in the longitudinal direction.

13. The absorbent article according to any one of claims 1 to 12,
wherein an inner edge (52IS) of the side slit extends outward in the width direction from the wide portion toward a rear side.

## Patentansprüche

1. Absorbierender Artikel (10), Folgendes umfassend:
eine Längsrichtung (L) und eine Breitenrichtung (W), die senkrecht zueinander sind;
einen Schrittbereich (S3), beinhaltend eine Mitte in Längsrichtung;
einen Vorderseitenbereich (S1), der an einer Vorderseite des Schrittbereichs positioniert ist, und einen Rückseitenbereich (S2), der an einer Rückseite des Schrittbereichs positioniert ist; und
einen absorbierenden Kern (50), der mindestens im Schrittbereich angeordnet ist,
wobei der absorbierende Kern ein Paar Seitenschlitze (52) aufweist, das so ausgebildet ist, dass es sich in Längsrichtung erstreckt, und der absorbierende Kern einen Mittelbereich (RC) umfasst, der durch die Seitenschlitze und Seitenbereiche (RS) eingeschlossen zwischen den Seitenschlitzen und den Außenkanten des absorbierenden Kerns positioniert ist,
wobei der Seitenschlitz einen breiten Abschnitt (53) umfasst, der eine Länge in der Breitenrichtung gleich oder größer als eine Lange in der Breitenrichtung des Seitenbereichs im Schrittbereich aufweist, und
wobei eine Mitte (53CL) des breiten Abschnitts in der Längsrichtung an einer Vorderseite von einer Mitte (10CL) des absorbierenden Artikels in der Längsrichtung positioniert ist.

2. Absorbierender Artikel nach Anspruch 1,
wobei eine Hinterkante (52R) des Seitenschlitzes innerhalb des Schrittbereichs positioniert ist.

3. Absorbierender Artikel nach Anspruch 1 oder 2,
wobei der Seitenschlitz in der Breitenrichtung innerhalb der Außenkante (50S) des absorbierenden Kerns positioniert ist.

4. Absorbierender Artikel nach einem der Ansprüche 1 bis 3, ferner Folgendes umfassend:
eine Decklage (41a), die an einer Hautoberflächenseite des absorbierenden Kerns positioniert ist; und
eine sich aufrichtende, leckageverhindernde Raffung (60), die an einer Hautoberflächenseite der Decklage positioniert ist,
wobei die leckageverhindernde Raffung einen Kontraktionsabschnitt (61), der durch Kontraktion eines leckageverhindernden elastischen Elements (66) aufgerichtet wird, und einen Basispunktabschnitt (62), der als Basispunkt des Anstiegs des Kontraktionsabschnitts dient, beinhaltet und
wobei der Basispunktabschnitt, der in Breitenrichtung außerhalb des Kontraktionsabschnitts positioniert ist, den Seitenbereich in einer Dickenrichtung (T) überlappt.

5. Absorbierender Artikel nach einem der Ansprüche 1 bis 4,
wobei ein in Längsrichtung kontrahierendes elastisches Beinelement (67) außerhalb des Seitenbereichs in Breitenrichtung bereitgestellt ist.

6. Absorbierender Artikel nach einem der Ansprüche 1 bis 5,
wobei auf einer virtuellen Linie (FL1), die sich in der Breitenrichtung über den breiten Abschnitt erstreckt, eine Länge des Seitenbereichs in der Breitenrichtung länger ist als eine Länge des Mittelbereichs in der Breitenrichtung.

7. Absorbierender Artikel nach einem der Ansprüche 1 bis 5,
wobei auf einer virtuellen Linie (FL1), die sich in der Breitenrichtung über den breiten Abschnitt erstreckt, eine Länge des Seitenbereichs in der Breitenrichtung gleich wie eine Länge des Mittelbereichs in der Breitenrichtung oder geringer als 1/2 dieser ist.

8. Absorbierender Artikel nach einem der Ansprüche 1 bis 7,
wobei der absorbierende Kern mit einem Mittelschlitz (55) bereitgestellt ist, der in Breitenrichtung zwischen den Seitenschlitzen positioniert ist und sich in der Längsrichtung erstreckt.

9. Absorbierender Artikel nach einem der Ansprüche 1 bis 8,
wobei der absorbierende Kern einen verengten Abschnitt (51) aufweist, dessen Länge in der Breitenrichtung kürzer ist als eine maximale Breite des absorbierenden Kerns, und wobei der verengte Abschnitt im Schrittbereich angeordnet ist.

10. Absorbierender Artikel nach Anspruch 9,
wobei eine Mitte (51GL) des verengten Abschnitts in der Längsrichtung an einer Vorderseite der Mitte des absorbierenden Artikels in der Längsrichtung positioniert ist.

11. Absorbierender Artikel nach Anspruch 9 oder 10,
wobei eine Vorderkante des Seitenschlitzes an einer Vorderseite einer Vorderkante des verengten Abschnitts positioniert ist und
eine Hinterkante (52R) des Seitenschlitzes an einer Vorderseite einer Hinterkante (51R) des verengten Abschnitts positioniert ist.

12. Absorbierender Artikel nach einem der Ansprüche 9 bis 11,
wobei der breite Abschnitt in einem Bereich angeordnet ist, in dem der verengte Abschnitt in der Längsrichtung bereitgestellt ist.

13. Absorbierender Artikel nach einem der Ansprüche 1 bis 12,
wobei sich eine Innenkante (52IS) des Seitenschlitzes in der Breitenrichtung von dem breiten Abschnitt nach außen zu einer Rückseite hin erstreckt.

## Revendications

1. Article absorbant (10) comprenant :
une direction longitudinale (L) et une direction de largeur (W) orthogonales l'une à l'autre ;
une région d'entrejambe (S3) comportant un centre dans la direction longitudinale ;
une région latérale avant (S1) positionnée sur un côté avant de la région d'entrejambe, et une région latérale arrière (S2) positionnée sur un côté arrière de la région d'entrejambe ; et
un noyau absorbant (50) disposé au moins dans la région d'entrejambe, dans lequel le noyau absorbant a une paire de fentes latérales (52) formées pour s'étendre dans la direction longitudinale, et le noyau absorbant comporte une région centrale (RC) prise en sandwich par les fentes latérales et des régions latérales (RS) positionnées entre les fentes latérales et des bords extérieurs du noyau absorbant,
dans lequel la fente latérale comporte une partie large (53) ayant une longueur dans la direction de largeur supérieure ou égale à une longueur dans la direction de largeur de la région latérale dans la région d'entrejambe, et
dans lequel un centre (53CL) de la partie large dans la direction longitudinale est positionné sur un côté depuis un centre (10CL) de l'article absorbant dans la direction longitudinale.

2. Article absorbant selon la revendication 1,
dans lequel un bord arrière (52R) de la fente latérale est positionné à l'intérieur de la région d'entrejambe.

3. Article absorbant selon la revendication 1 ou 2,
dans lequel la fente latérale est positionnée à l'intérieur du bord extérieur (50S) du noyau absorbant dans la direction de largeur.

4. Article absorbant selon l'une quelconque des revendications 1 à 3, comprenant en outre :
une feuille supérieure (41a) positionnée sur un côté de surface de la peau du noyau absorbant ; et
une fronce anti-fuite redressée (60) positionné sur un côté de surface de la peau de la feuille supérieure,
dans lequel la fronce anti-fuite comporte une partie de contraction (61) redressée par une contraction d'un élément élastique anti-fuite (66) et d'une partie de point de base (62) servant de point de base d'élévation de la partie de contraction, et
dans lequel la partie de point de base positionnée à l'extérieur de la partie de contraction dans la direction de largeur chevauche la région latérale dans une direction d'épaisseur (T).

5. Article absorbant selon l'une, quelconque des revendications 1 à 4,
dans lequel un élément élastique de jambe (67) se contractant dans la direction longitudinale est prévu à l'extérieur de la région latérale dans la direction de largeur.

6. Article absorbant selon l'une quelconque des revendications 1 à 5,
dans lequel sur une ligne virtuelle (FL1) s'étendant dans la direction de largeur à travers la partie large, une longueur de la région latérale dans la direction de largeur est plus longue qu'une longueur de la région centrale dans la direction de largeur.

7. Article absorbant selon l'une quelconque des revendications 1 à 5,
dans lequel sur une ligne virtuelle (FL1) s'étendant dans la direction de largeur à travers la partie large, une longueur de la région latérale dans la direction de largeur est inférieure ou égale à 1/2 d'une longueur de la région centrale dans la direction de largeur.

8. Article absorbant selon l'une quelconque des revendications 1 à 7,
dans lequel le noyau absorbant est pourvu d'une fente centrale (55) positionnée entre les fentes latérales dans la direction de largeur et s'étendant dans la direction longitudinale.

9. Article absorbant selon l'une quelconque des revendications 1 à 8,
dans lequel le noyau absorbant comporte une partie rétrécie (51) ayant une longueur dans la direction de largeur plus courte qu'une largeur maximale du noyau absorbant, et dans lequel la partie rétrécie est disposée dans la région d'entrejambe.

10. Article absorbant selon la revendication 9,
dans lequel un centre (51CL) de la partie rétrécie dans la direction longitudinale est positionné sur un côté avant du centre de l'article absorbant dans la direction longitudinale.

11. Article absorbant selon la revendication 9 ou 10,
dans lequel un bord avant de la fente latérale est positionné sur un côté avant d'un bord avant de la partie rétrécie, et
un bord arrière (52R) de la fente latérale est positionné sur un côté avant d'un bord arrière (51R) de la partie rétrécie.

12. Article absorbant selon l'une quelconque des revendications 9 à 11,
dans lequel la partie large est disposée dans une région où la partie rétrécie est prévue dans la direction longitudinale.

13. Article absorbant selon l'une quelconque des revendications 1 à 12,
dans lequel un bord intérieur (52IS) de la fente latérale s'étend vers l'extérieur dans la direction de largeur depuis la partie large vers un côté arrière.
